# EUROPEAN PATENT SPECIFICATION

(11) **EP 0 554 776 B1**
(45) Date of publication and mention of the grant of the patent: **28.05.1997**
(21) Application number: 93101198.5
(22) Date of filing: 27.01.1993
(51) Int. Cl.: C09B 67/02, C09C 1/00

(54) **Novel flaky pigments**
Neuartige Schuppenpigmente
Nouveaux pigments en écailles

(30) Priority: 06.02.1992 JP 65470/92
(43) Date of publication of application: 11.08.1993
(73) Proprietor: MERCK PATENT GmbH, D-64271 Darmstadt (DE)
(72) Inventor: Yazawa, Masahiko, Iwaki-shi, Fukushima-ken (JP); Noguchi, Tamio, Iwaki-shi, Fukushima-ken (JP)

(56) References cited:
- EP-A- 0 218 156
- EP-A- 0 220 617
- EP-A- 0 278 633
- US-A- 3 582 382

## Description

The present invention relates to a pigment having good chroma and transparency, which may be used in cosmetics, top coating paints for cars, plastic colorants, printing inks, paints for domestic electric appliances, paints for building materials and urushi (Japanese lacquer) paints, as well as to a method of preparing the same.

More precisely, the present invention relates to a flaky pigment composed of flaky substrates as uniformly and firmly coated with fine pigment and/or dye particles, as well as to a method of preparing the same.

### [Prior Art]

Where pigments and dyes are used in various products as a colorant, it has heretofore been known to necessarily carry out a pre-treatment of dyes and pigments with an atomizer, roll mill or sand mill, for the purpose of grinding the aggregated pigments and dyes to thereby stabilize coloration with them. In such a conventional method, however, selection of the pre-treatment condition is difficult since the respective pigments and dyes have different cohesive forces. In addition, since some pigments and dyes could not have a sufficient dispersion stability, re-aggregation of the pulverized particles would occur. Anyhow, the conventional method has a drawback of not yielding good coloration because of the reasons.

As a technique of overcoming the drawback of the prior art, for example, Japanese Patent Application Laid-Open No. 62-91565 has proposed a method of obtaining an well-coloring pigment material by adding a finely powdered flaky substrate to a dye which has been formed into an aqueous solution thereof by a chemical means, followed by precipitating and depositing dye particles on the surfaces of the substrates.

Japanese Patent Application Laid-Open No. 63-243168 has proposed formation of a colored mica pigment by precipitating pigment particles on the surfaces of substrates in an aqueous system so as to cover the substrates with the precipitated pigment.

US Patent No. 3582382 discloses a nacreous pigment comprising a translucent layer of titanium dioxide deposited on a translucent flake substrate, such as mica, is made by continuously contacting the substrate particles with titanium tetrachloride vapor at atmospheric pressure, followed by continuously hydrolyzing the deposited titanium tetrachloride to form titanium dioxide.
European Patent No. 0220617 discloses flaky coloured pigments comprising a flaky substrate the surface of which is covered by a finely divided colour pigment adhering uniformly to the flaky substrate by the use of an organic big molecular compound as a binder. The pigment is produced by suspending the flaky substrate in a dispersion or a solution of a finely divided colour pigment, by mixing the resulting suspension with a solution of an organic high molecular compound and precipitating the colour pigment and the organic high molecular compound onto the flaky substrate.

However since the improved methods in the prior art are so-called wet methods in which the treatment is effected in a liquid medium, they need filtration and drying steps so that the running cost for manufacturing the product is high. In addition, since dispersion of fine particles of pigments and dyes is often bad, good coloration could not be attained. Thus, the methods still have such drawbacks. Moreover, the pigment or dye particles which nave not adhered to the surfaces of the substrates or hardly adhering pigment or dye particles in the pre-treatment step would be separated or washed out during the subsequent filtering step or washing step. In the case, an intended sufficient coloration would not be attained in proportion to the amount of the dye or pigment used.

The present inventors variously studied for the purpose of obtaining a method of preparing a flaky pigment as coated with pigment and/or dye particles which may be carried out at a low running cost, which is simple and which may yield a flaky pigment having an excellent coloring capacity. As a result, they have succeeded in attaining a novel improved method of producing a flaky pigment having a better coloring capacity than any other conventional pigment materials and in providing a novel flaky pigment to be obtained by the method.

### [Disclosure of the Invention]

The present inventors have found that when flaky substrates and pigment and/or dye particles are subjected to high-speed stirring for a determined period of time in the absence of a liquid medium for the purpose of coating the surfaces of the flaky substrates with the pigment and/or dye particles, the flaky substrates and the pigment and/or dye particles may form an ordered mixture (reference: Planning of Fine Particles (published by Industrial Search Association, Japan), pages 148 to 149) so that a flaky pigment having excellent chroma and dispersibility and having an excellent adhesion stability of the pigment and/or dye particles to the flaky substrates can be obtained. The present invention has been attained on the basis of the finding.

In accordance with the present invention, therefore, there is provided a flaky pigment having a mean particle size of from 5 to 60 µm, which is in the form of composite particles based on an ordered mixture, the composite particles being formed by subjecting a mixture composed of a flaky substrate comprising flaky substrates each having an aspect ratio of from 10 to 120 and a pigment and/or a dye comprising particles having a mean particle size of 5 µm or less, to high-speed stirring treatment in the absence of a liquid medium.

In general, formation of one particle by bonding two or more particles to each other is referred to as "compositation". The above-mentioned composite of the present invention means a composite of plural particles as formed by such compositation. In such compositation of a powder/powder system, a mixed state where fine particles have adhered to the surfaces of other fine particles is called an "ordered mixture" (reference: Planning of Fine particles (written by Masumi Koishi and published by Industrial Search Association, Japan).

The base to be used in the present invention is a flaky substance which is suitable to flaky pigment particles Examples thereof include metal oxide-coated mica, mica, sericite, kaolin, etc. The flaky substrate to be used in the present invention is composed of particles each having an aspect ratio of from 10 to 120 and having a mean particle size of from 5 to 60 µm.

If the flaky substrate particles have an aspect ratio of less than 10, dispersion of fine particles of the pigment and/or dye with them could not be effected sufficiently so that the flaky pigment to be obtained could not have a sufficient coloring capacity. On the contrary, if they have an aspect ratio of more than 120, retention of the shape of the substrates would be difficult since the mechanical strength of them to the stirring energy is insufficient.

The pigment and/or dye particles to be used for coating the flaky substrates in the present invention have a mean particle size of 5 µm or less. If they have a a larger particle size than the defined value, the coloring capacity of the flaky pigment to be obtained would be poor.

Where the flaky pigment of the present invention is used in cosmetics, examples of the above-mentioned coating pigments include azo pigments such as Pigment Red 57-1, Pigment Red 3, Pigment Yellow 1, etc.; anthraquinone pigments such as Vat Blue 6, etc.; indigo pigments such as Vat Blue 1, etc.; thioindigo pigments such as Vat Red 1, etc.; and phthalocyanine pigments such as Pigment Blue 15, etc. As examples of the coating dyes in the case, there are mentioned azo dyes such as Acid Yellow 40, Solvent Yellow 5, etc.; nitroso dyes such as Acid Green 1, etc.; triphenylmethane dyes such as Acid Blue 5, etc.; xanthene dyes such as Acid Red 51, Solvent Red 92, Basic Violet 10, etc.; quinoline dyes such as Acid Yellow 3, Solvent Yellow 33, etc.; and indigo dyes such as Acid Green 25, Acid Blue 74, etc.

Where the flaky pigment of the present invention is used in coating paints for cars, higher pigments having excellent light resistance and weather resistance are used as the above-mentioned coating pigments. For instance, there are mentioned azomethine pigments such as Pigment Yellow 117, etc.; quinophthalone pigments such as Pigment Yellow 138, etc.; isoindolinone pigments such as Pigment Yellow 139, Pigment Orange 66, Pigment Red 257, etc.; condensed azo pigments such as Pigment Yellow 128, Pigment Brown 23, etc.; flavanthrone pigments such as Pigment Yellow 24, etc.; perinone pigments such as Pigment Orange 43, etc.; quinacridone pigments such as Pigment Orange 48, Pigment Red 122, Pigment Red 206, Pigment Red 207, Pigment Violet 19, etc.; pyranthrone pigments such as Pigment Orange 51, Pigment Red 216, Pigment Red 226, etc.; thioindigo pigments such as Pigment Red 88, etc.; naphthol AS pigments such as Pigment Red 170, Pigment Red 253, etc,; perylene pigments such as Pigment Red 179, Pigment Violet 29, Pigment Brown 26, etc.; anthraquinone pigments such as Pigment Red 168, etc.; dioxazine pigments such as Pigment Violet 23, etc.; phthalocyanine pigments such as Pigment Blue 15:1 to 6, etc.; and indanethrene pigments such as Pigment Blue 60, etc.

Where the flaky pigment of the present invention is used in coating paints for general use, coating paints for coloration of plastics or printing inks, examples of the above-mentioned coating pigments include azo pigments such as Pigment Red 3, Pigment Red 5, Pigment Yellow 14, Pigment Yellow 83, Pigment Yellow 95, etc.; phthalocyanine pigments such as Pigment Blue 15:1 to 6, etc.; threne pigments such as Vat Yellow 20, Vat Orange 3, Pigment Red 177, Vat Blue 4, etc.; indigo pigments such as Pigment Red 88, etc.; perinone pigments such as Pigment Orange 43, etc.; perylene pigments such as Pigment Red 123, Pigment Red 178, etc.; phthalone pigments such as Pigment Yellow 138, etc.; dioxazine pigments such as Pigment Violet 23, etc.; quinacridone pigments such as Pigment Violet 19, Pigment Red 122, etc.; and isoindolinone pigments such as Pigment Yellow 109, Pigment Yellow 110, etc.

Where the flaky pigment of the present invention is used in coating paints for general use, coating paints for coloration of plastics or printing inks, examples of the above-mentioned coating dyes include azo dyes such as Acid Black 1, Direct Red 28, Direct Green 28, Disperse Blue 79, Basic Red 18, etc.; anthraquinone dyes such as Acid Blue 78, Disperse Blue 60, Vat Blue 4, etc.; indigo dyes such as Vat Blue 1, Vat Red 1, etc.; phthalocyanine dyes such as copper phthalocyanine, etc.; xanthene dyes such as Acid Red 94, etc.; thiazine dyes such as Basic Blue 9, etc.; and reactive dyes such as Reactive Red 6, Reactive Yellow 3, Reactive Blue 19, Reactive Black 5, etc.

These pigments and dyes are desired to be pre-ground prior to be applied to flaky substrates for coating them.

The energy for coating the pigment and/or dye grains over the surfaces of flaky substrates is defined to be 290 J or more, preferably 390 J or more, per unit gram of the substance to be treated.

If the energy is defined to be less than 290 J per unit gram, the frequency of contact of the particles to each other would be small because the energy to be imparted thereto by stirring is insufficient, so that aggregates of pigment and/or dye particles would be formed and the flaky pigment to be formed could not have a sufficient coloring capacity.

The stirring speed in the step of coating pigment and/or dye particles over the surfaces of flaky substrate particles is defined to be 30 m/sec or more, preferably from 40 to 70 m/sec, as the rotating speed of the stirring blade, whereby the intended flaky pigment of the present invention can be obtained.

If the rotating speed is less than 30 m/sec, the frequency of contact of the particles to each other would be small so that aggregates of pigment and/or dye particles would remain and the flaky pigment to be formed could not have a sufficient coloring capacity.

The type of the stirring reactor and the stirring system with it are not specifically defined, but a batch system mixer is suitable.

The mixing reactor to be used in preparing the flaky pigment of the present invention is needed to be able to impart an energy of 25 J or more, preferably 33 J or more, per unit cm³ to the capacity of the reactor chamber and to be equipped with stirrer(s) capable of mixing all the substances as being treated by convection stirring. In addition, in feeding the substances to be treated into the reactor, it is desired that a mixture of pigment and/or dye particles and flaky substrates is fed thereinto in an amount of 70 % or less, preferably from 20 to 70 %, of the capacity of the reactor vessel.

If the energy amount per unit cm³ is less than 25 J, the frequency of contact of the particles to each other would be small because the energy to be imparted thereto by stirring is insufficient, so that aggregates of pigment and/or dye particles would remain and the flaky pigment to be formed could not have a sufficient coloring capacity.

If the amount of the substances as fed into the reactor is too small, the small amount would be underloaded to the power of the stirrer to cause racing of the stirrer. In the case, the motor, shaft and bearing of the stirrer would unfavorably be broken. On the other hand, if the amount is more than 70 %, the large amount would be overloaded to the power of the stirrer to cause breakage of the above-mentioned parts of the stirrer, or coloration of the dye and/or pigment as incorporated would be poor since a sufficient convention motion could not be imparted to the substances as being treated.

### [Effect of the Invention]

In the present invention, although the mechanism of coating pigment and/or dye particles over the surfaces of flaky substrates by high-speed stirring is not sufficiently clarified, it may be considered that the stirring energy of high-speed stirring could function as a collision force, a compression force, a shearing force and a heat-generating factor to yield an effect of finely pulverizing pigment and/or dye particles and of fixing the pulverized pigment and/or dye particles over the surfaces of flaky substrates.

### [Examples]

Next, the present invention will be explained in more detail by way of the following examples, which, however, are not intended to restrict the scope of the present invention.

### [Example 1]

100 g of a mica having a mean aspect ratio of of 10 and a mean particle size of 5 µm and 2.56 g of an organic pigment Red 226 were put in a mixer having a capacity of 1200 ml (high-speed blender mixer, manufactured by Warling Co.) (the total charge percentage being 30 %) and were stirred and blended therein with a stirring energy of 84.2 kJ and a stirring speed of 70 m/sec as a rotating speed, to obtain a pigment having a high chroma. By observation with a scanning electromicroscope, it was confirmed that the particles of the organic pigment Red 226 uniformly adhered to the surfaces of the particles of the pigment.

The pigment thus obtained had a high chroma and had a good dispersibility, and adhesion of the organic pigment particles to the substrates was good.

### [Example 2]

100 g of a mica having a mean aspect ratio of 80 and a mean particle size of 40 µm and 2.56 g of an organic pigment Red 226 were put into the same mixer as that used in Example 1 (the total charge percentage being 25 %) and were stirred and blended therein with a stirring energy of 84.2 kJ and a stirring speed of 70 m/sec as a rotating speed, to obtain a pigment having a high chroma. By observation with a scanning electromicroscope, it was confirmed that the particles of the organic pigment Red 226 uniformly adhered to the surfaces of the particle of the pigment.

The pigment thus obtained had a high chroma and had a good dispersibility, and adhesion of the organic pigment particles to the substrates was good.

### [Example 3]

100 g of a titanium oxide-coated mica having a mean aspect ratio of 44 and a mean particle size of 22 µm and 2.56 g of an organic pigment Red 226 were put into the same mixer as that used in Example 1 (the total charge percentage being 28 %) and were stirred and blended therein with a stirring energy of 84.2 kJ and a stirring speed of 70 m/sec as a rotating speed, to obtain a pigment having a high chroma. By observation with a scanning electromicroscope, it was confirmed that the particles of the organic pigment Red 226 uniformly adhered to the surfaces of the particles of the pigment.

The pigment thus obtained had a high chroma and had a good dispersibility, and adhesion of the organic pigment grains to the substrates was good.

### [Example 4]

The same process as in Example 3 was repeated except that the stirring and blending was effected with a stirring energy of 40.1 kJ and a stirring speed of 40 m/sec as a rotating speed, to obtain a pigment having a high chroma. By observation with a scanning electromicroscope, it was confirmed that the particles of the organic pigment Red 226 uniformly adhered to the surfaces of the particles of the pigment.

The pigment thus obtained had a high chroma and had a good dispersibility, and adhesion of the organic pigment particles to the substrates was good.

### [Example 5]

100 g of a silver pearly luster pigment comprising a titanium oxide-coated mica having a mean aspect ratio of 44 and a mean particle size of 22 µm and 20 g of an organic pigment Red 226 were put into the same mixer as that used in Example 1 (the total charge percentage being 40 %) and were stirred and blended therein with a stirring energy of 84.2 kJ and a stirring speed of 70 m/sec as a rotating speed, to obtain a pigment having a high chroma. By observation with a scanning electromicroscope, it was confirmed that the particles of the organic pigment Red 226 uniformly adhered to the surfaces of the particles of the pigment.

The pigment thus obtained had a high chroma and had a good dispersibility, and adhesion of the organic pigment particles to the substrates was good.

### [Example 6]

100 g of a titanium oxide-coated mica having a mean aspect ratio of 44 and a mean particle size of 22 µm and 2.56 g of an organic pigment Blue 404 were put into the same mixer as that used in Example 1 (the total charge percentage being 28 %) and were stirred and blended therein with a stirring energy of 84.2 kJ and a stirring speed of 70 m/sec as a rotating speed, to obtain a pigment having a high chroma. By observation with a scanning electromicroscope, it was confirmed that the particles of the organic pigment Blue 404 uniformly adhered to the surfaces of the particles of the pigment.

The pigment thus obtained had a high chroma and had a good dispersibility, and adhesion of the organic pigment particles to the substrates was good.

### [Example 7]

100 g of a titanium oxide-coated mica having a mean aspect ratio of 44 and a mean particle size of 22 µm and 2.56 g of an organic pigment yellow 401 were put into the same mixer as that used in Example 1 (the total charge percentage being 28 %) and were stirred and blended therein with a stirring energy of 84.2 kJ and a stirring speed of 70 m/sec as a rotating speed, to obtain a pigment having a high chroma. By observation with a scanning electromicroscope, it was confirmed that the particles of the organic pigment yellow 401 uniformly adhered to the surfaces of the particles of the pigment.

The pigment thus obtained had a high chroma and had a good dispersibility, and adhesion of the organic pigment particles to the substrates was good.

### [Example 8]

100 g of a titanium oxide-coated mica having a mean aspect ratio of 44 and a mean particle size of 22 µm and 2.56 g of a phthalocyanine higher organic pigment for top coat paint for cars, G-314 (trade name by Sanyo Dye Co., Japan) were put into the same mixer as that used in Example 1 (the total charge percentage being 28 %) and were stirred and blended therein with a stirring energy of 84.2 kJ and a stirring speed of 70 m/sec as a rotating speed, to obtain a pigment having a high chroma. By observation with a scanning electromicroscope, it was confirmed that the particles of the blue pigment G-314 uniformly adhered to the surfaces of the particles of the pigment.

The pigment thus obtained had a high chroma and had a good dispersibility, and adhesion of the organic pigment particles to the substrates was good.

### [Comparative Example 1]

100g of a kaolin having a mean aspect ratio of 5 and a mean particle size of 2 µm (ASP-170, trade name by Fuji Talc Co., Japan) and 2.56 g of an organic pigment Red 226 were put into the same mixer as that used in Example 1 (the total charge percentage being 40 %) and were stirred and blended therein with a stirring energy of 84.2 kJ and a stirring speed of 70 m/sec as a rotating speed. However, the resulting product was found to contain aggregates of the organic pigment particles.

### [Comparative Example 2]

100g of a titanium oxide-coated mica having a mean aspect ratio of 44 and a mean particle size of 22 µm and 2.56 g of an organic pigment Red 226 were put into the same mixer as that used in Example 1 (the total charge percentage being 28 %) and were stirred and blended therein with a stirring energy of 20.7 kJ and a stirring speed of 20 m/sec as a rotating speed. However, the resulting product was found to contain aggregates of the organic pigment particles.

### [Comparative Example 3]

280g of a titanium oxide-coated mica having a mean aspect ratio of 44 and a mean particle size of 22 µm and 7.18 g of an organic pigment Red 226 were put into the same mixer as that used in Example 1 (the total charge percentage being 80 %) and were stirred and blended therein with a stirring energy of 236 kJ and a stirring speed of 70 m/sec as a rotating speed. However, the resulting product was found to contain aggregates of the organic pigment particles.

### [Comparative Example 4]

400g of organic pigment Red 226 , 550 g of an aqueous potassium hydroxide solution having a concentration of 40° Baumé and one liter of an aqueous sodium thiosulfate solution having a concentration of 200 g/liter were added to 10 liters of a deionized water having a temperature of 95°C and stirred therein to prepare a colloidal dispersion of a vat dye. 2000 g of the same silver pearly luster pigment as that used in Example 5 was added and dispersed in the colloidal dispersion, which was then subjected to aerial oxidation with stirring at 80°C. After 15 minutes, 800 g of 15 wt.% hydrogen peroxide was added thereto at a speed of about 20 ml/min and stirred for 10 minutes at 85°C. Afterwards, about 600 g of 35 % hydrochloric acid was added thereto at a temperature of 70°C, whereby the reaction system was adjusted to have pH of 5.0. After 2 hours, the product was taken out by filtration and washed. This was dried at 110°C for 20 hours and then at 145°C for 4 hours to obtain a red-colored pigment.

The organic pigment-coated flaky pigment samples as obtained in Examples 1 to 5 and Comparative Examples 1 to 4 were subjected to the following tests to evaluate the properties of them. The results obtained are shown in Table 1 below.
① Visual test of determining presence or absence of aggregates of organic pigment particles.
   0.5 g of a sample to be tested was dispersed in 4.5 g of VS Medium (trade name, by Dainichi Seika Co., Japan), and the resulting dispersion was spread on a coated paper and dried thereon, whereupon presence or absence of aggregates of organic pigment particles in the dried powder was visually inspected.
② Test of determining the colored degree of organic pigment: For the powdered sample on the coated paper as prepared in ①, a value (45°/0° measuring angle) was measured by Hunter system. The higher the a value of the tested sample was, the the more effectively colored the organic pigment Red 226 was.
③ Test of determining the adhesion power of organic pigment particles to substrates :

0.1 g of an organic pigment-coated flaky pigment sample was dispersed in 1.9 g of a liquid paraffin 350S (trade name, by Chuoh Chemical Co., Japan), and a shear stress was imparted thereto in a Hoover muller (manufactured by Yoshimitsu Seiki Co., Japan) under the condition of no load and 50 rotations, whereupon the bleeding condition of the dye before and after the Hoover muller test was visually inspected with an optical microscope.

### [Advantage of the Invention]

In accordance with the method of the present invention, the stirring energy to be derived from the high-speed stirring acts as a collision force, a compression force, a shearing force and a heat-generating factor to display an excellent effect for finely pulverizing and powdering pigment and/or dye particles and for compositating the resulting pigment and/or dye particles with flaky substrates which are harder than the pigment and/or dye particles. Accordingly, the composite flaky pigment thus formed by the method of the present invention is free from lowering of the coloring capacity, and the method of the present invention is free from elevation of the manufacture cost which is inevitable in the conventional wet method. In accordance with the present invention, therefore, a flaky pigment which is excellent in the coloring capacity, the dispersibility and the adhesiveness between the pigment and dye particles and the flaky substrates can be produced at a low manufacture cost.

Where the pigment as claimed in claim 1 of the present invention is used as a pigment material for cosmetics, it displays excellent effects of improving the chroma, preventing the white blur and improving the uniform dispersion of the pigment particles. Where the pigment is used as a pigment material in a top coat paint for cars, it displays excellent effects of improving the chroma and preventing the white blur of the undertone. Where it is used as a pigment material for coloring plastics, it displays excellent effects of improving the chroma and preventing the plate-out. Where it is used as a pigment material in printing inks or in coating paints for general use, it displays excellent effects of improving the chroma, improving the uniform dispersibility and preventing the bleeding.

### [Use Examples]

The flaky pigment of the present invention can be used as a pigment material in cosmetics, a pigment material in top coat paints for cars, a pigment material for coloration of plastics, a pigment material in printing inks, a pigment material in coating paints for domestic electric appliances, a pigment material in coating paints for building parts, a pigment material in urushi (Japanese lacquer) paints.

### Application Example 1:

This illustrates embodiments of using the flaky pigment of the present invention as a pigment material in cosmetics.
(1) Lipstick: 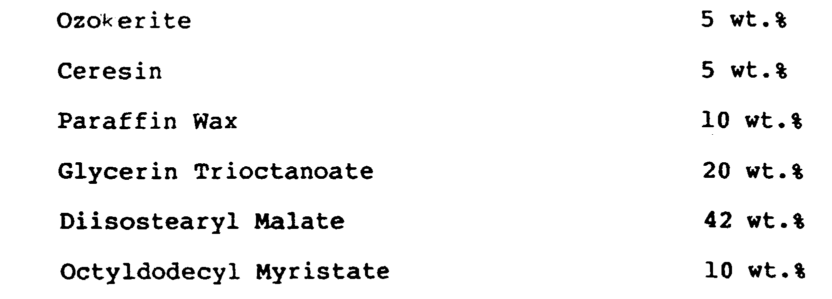 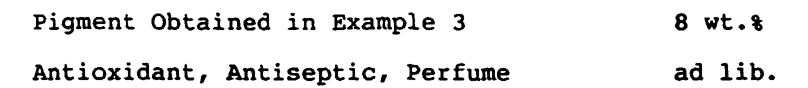
Using the above-mentioned components, lipsticks were prepared.
(2) Compact Cake/Eye Shadow:

| | |
|---|---|
| Talc | 38 wt.% |
| Mica | 5 wt.% |
| Magnesium Stearate | 3 wt.% |
| Nylon Powder | 8 wt.% |
| Pigment Obtained in Example 3 | 35 wt.% |
| Red Iron Oxide | 2 wt.% |
| Liquid Paraffin | 5 wt.% |
| Isopropyl Myristate | 4 wt.% |
| Antioxidant, Antiseptic | ad lib. |

Using the above-mentioned components, compact cakes/eye shadow cakes were prepared.
(3) Nail Lacquer:

| | |
|---|---|
| Nitrocellulose | 15 wt.% |
| Alkyd Resin | 12 wt.% |
| Butyl Phthalate | 6 wt.% |
| Butyl Acetate | 23 wt.% |
| Ethyl Acetate | 25 wt.% |
| Ethanol | 7 wt.% |
| Toluene | 5 wt.% |
| Pigment Obtained in Example 3 | 5 wt.% |
| Organic Bentonite | 2 wt.% |

Using the above-mentioned components, a nail lacquer was prepared.

### Use Example 2:

Where the flaky pigment of the present invention is used as a pigment material in a top coat paint for cars, it is incorporated into the top coat layer in combination with a carbon black pigment, a metal powder such as aluminium powder and a pearly pigment containing mica as a substrate. Alternatively, it may be incorporated into a top coat resin singly in an amount of from 0.1 to 50 % by weight. One embodiment of the case is illustrated below.

| | |
|---|---|
| A. Acryl-Melamine Resin | 100 wt.pts. |
| Composition of A: | |
| Acrydec 47-712 | (70 wt.%) |
| Superbeckamine G821-60 | (30 wt.%) |
| B. Pigment Obtained in Example 8 | 20 wt.pts. |
| C. Acryl-Melamine Resin Thinner | |
| Composition of C: | |
| Ethyl Acetate | (50 wt.pts.) |
| Toluene | (30 wt.pts.) |
| N-butanol | (10 wt.pts.) |
| Solbesso #150 | (40 wt.pts.) |

A and B were blended, and C was added to the resulting blend so as to thin it to have a viscosity suitable for spray-coating (from 12 to 15 seconds with Fordcup #4). This was coated on a substrate by spray-coating to form a base coat layer thereon.

### Use Example 3:

This illustrates an embodiment of using the flaky pigment of the present invention as a pigment for coloration of a plastic material.

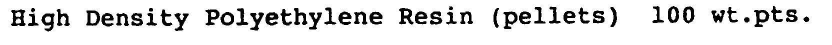

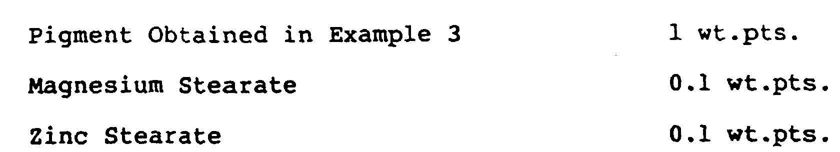

Pellets having the composition mentioned above were subjected to injection molding through an injection molding machine.

### Use Example 4:

This illustrates an embodiment of using the flaky pigment of the present invention as a pigment material for a printing ink.

| | |
|---|---|
| CCST Medium (nitrocellulose resin, manufactured by Toyo Ink Co., Japan) | 40 wt.pts. |
| Pigment Obtained in Example 3 | 8 wt.pts. |

A solvent NC 102 (manufactured by Toyo Ink Co.) was added to the composition mentioned above, which was adjusted to have a viscosity of 20 sec (with Zancup No. 3). The ink prepared was used for printing.

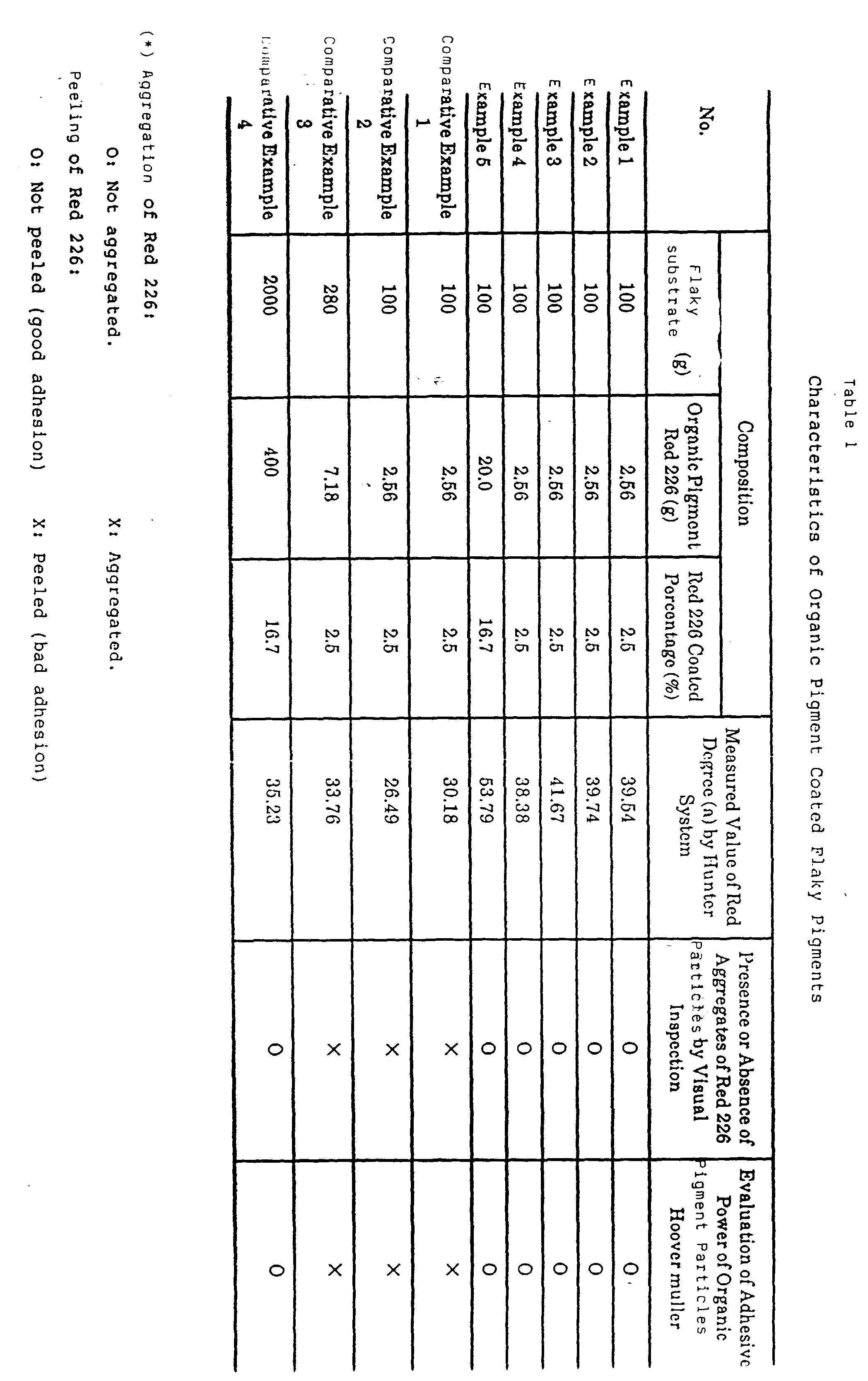

## Claims

1. A flaky pigment having a mean particle size of from 5 to 60 µm, which is in the form of composite particles based on an ordered mixture, the composite particles being obtainable by subjecting a mixture composed of a flaky substrate comprising flaky substrates each having an aspect ratio of from 10 to 120 and a pigment and/or a dye comprising particles having a mean particle size of 5 µm or less, to high speed stirring treatment in the absence of a liquid medium.

2. A method of preparing a flaky pigment as claimed in claim 1, in which a stirring energy of 290 J or more per unit gram is imparted to the mixture particles comprising the said pigment and/or dye and the said flaky substrate so that the particles are formed into composite particles in a high-speed gaseous stream in the absence of a liquid medium.

3. The method of preparing a flaky pigment as claimed in claim 2, in which the said mixture is subjected to high-speed stirring treatment at a stirring speed of 30 m/sec or more as a rotating speed.

4. The method of preparing a flaky pigment as claimed in claim 3, in which an appparatus as equipped with stirrer(s) and stirring blade(s) capable of imparting an energy of 25 J or more per unit cm³ to the capacity of the reactor and a sufficient convection mixing action to the said mixture is used.

5. The method of preparing a flaky pigment as claimed in claim 4, in which the said mixture is fed into the vessel in an amount of 70% or less of the capacity of the reactor vessel, and is treated with high shear rate.

6. Use of a flaky pigment as claimed in claim 1 in pigment coloration of cosmetics, top coating paints for cars, plastics, printing inks, paints for domestic electric appliances, paints for building materials and urushi (Japanese lacquer) paints.

## Patentansprüche

1. Schuppenförmiges Pigment mit einer mittleren Teilchengröße von 5 bis 60 µm, das in Form von Verbundteilchen aus einer geordneten Mischung vorliegt, wobei die Verbundteilchen dadurch erhältlich sind, daß man eine Mischung aus einem schuppenförmigen Substrat, enthaltend schuppenförmige Substrate mit je einem Verhältnis der Ausdehnung in der Hauptdimension zur Dicke von 10 bis 120, und ein Pigment und/oder einen Farbstoff enthaltenden Teilchen mit einer mittleren Teilchengröße von höchstens 5 µm in Abwesenheit eines flüssigen Mediums hochtourig verrührt.

2. Verfahren zur Herstellung eines schuppenförmigen Pigments gemäß Anspruch 1, bei dem man auf die Mischungsteilchen, enthaltend das Pigment und/oder den Farbstoff und das schuppenförmige Substrat, eine Rührenergie von mindestens 290 J pro Gramm ausübt, so daß sich die Teilchen in einem Hochgeschwindigkeitsgasstrom in Abwesenheit eines flüssigen Mediums zu Verbundteilchen verbinden.

3. Verfahren zur Herstellung eines schuppenförmigen Pigments nach Anspruch 2, bei dem man die Mischung mit einer Rührgeschwindigkeit von mindestens 30 m/sec als Drehgeschwindigkeit verrührt.

4. Verfahren zur Herstellung eines schuppenförmigen Pigments nach Anspruch 3, bei dem man eine Vorrichtung einsetzt, die mit einem oder mehreren Rührwerken und einer oder mehreren Rührschaufeln ausgerüstet ist, die in der Lage sind, auf das Reaktorvolumen eine Energie von mindestens 25 J pro cm³ und auf die Mischung eine ausreichende Konvektionsmischwirkung auszuüben.

5. Verfahren zur Herstellung eines schuppenförmigen Pigments nach Anspruch 4, bei dem man die Mischung in einer Menge, die höchstens 70% des Reaktorvolumens entspricht, einfüllt und hochscherend behandelt.

6. Verwendung eines schuppenförmigen Pigments gemäß Anspruch 1 bei der Einfärbung von Kosmetika, Autodecklacken, Kunststoffen, Druckfarben, Lacken für elektrische Haushaltsgeräte, Lacken für Baustoffe und Japanlacken (Urushi).

## Revendications

1. Pigment floconneux ayant une taille particulaire moyenne de 5 à 60 µm qui se présente sous la forme de particules composites à base d'un mélange ordonné, les particules composites, pouvant être obtenues en soumettant le mélange composé d'un substrat floconneux comprenant des substrats floconneux, dont chacun a un indice de forme de 10 à 120, et un pigment et/ou un colorant comprenant des particules ayant une taille particulaire moyenne de 5 µm, ou moins, destiné à un traitement sous agitation à vitesse élevée en l'absence d'un milieu liquide.

2. Procédé de préparation d'un pigment floconneux, selon la revendication 1, dans lequel on communique une énergie d'agitation de 290 J, ou plus, par gramme aux particules du mélange comprenant ledit pigment et/ou ledit colorant ainsi que ledit substrat floconneux de sorte que les particules se transforment en particules composites dans un flux gazeux à vitesse élevée en l'absence d'un milieu liquide.

3. Procédé de préparation d'un pigment floconneux selon la revendication 2, dans lequel on soumet ledit mélange à un traitement sous agitation à vitesse élevée à une vitesse d'agitation de 30 m/s, ou plus, comme vitesse de rotation.

4. Procédé de préparation d'un pigment floconneux selon la revendication 3, dans lequel on utilise un appareil tel qu'équipé d'un ou de plusieurs agitateurs et d'une ou de plusieurs pales d'agitation aptes à communiquer une énergie de 25 J, ou plus, par cm³ au contenu du réacteur et dans lequel on soumet ledit mélange à une action de mélange par convection suffisante.

5. Procédé de préparation d'un pigment floconneux selon la revendication 4, dans lequel on introduit ledit mélange dans la cuve en une quantité de 70 %, ou moins, du contenu de la cuve du réacteur et dans lequel on traite celui-ci avec un gradient de cisaillement élevé.

6. Utilisation d'un pigment floconneux selon la revendication 1 dans la coloration à base de pigments de cosmétiques, de peintures pour couche de finition de voitures, de matières plastiques, encres d'impression, peintures pour dispositifs électriques à usage domestique, peintures pour matériaux de construction et peintures urushi (laque japonaise).
